Europäisches Patentamt

European Patent Office

Office européen des brevets

(11) Publication number: **0 265 536**
**A1**

## EUROPEAN PATENT APPLICATION

(21) Application number: **86114865.8**

(22) Date of filing: **27.10.86**

(51) Int. Cl.⁴ **A61M 29/02**

(43) Date of publication of application:
**04.05.88 Bulletin 88/18**

(84) Designated Contracting States:
**DE FR GB SE**

(71) Applicant: **Marinoni, Elda**
**Viale Albini 6**
**I-24100 Bergamo(IT)**

Applicant: **Magnasco, Dante**
**Via Brosetta 81/A**
**I-24100 Bergamo(IT)**

(72) Inventor: **Marinoni, Elda**
**Viale Albini 6**
**I-24100 Bergamo(IT)**
Inventor: **Magnasco, Dante**
**Via Brosetta 81/A**
**I-24100 Bergamo(IT)**

(74) Representative: **Modiano, Guido et al**
**MODIANO, JOSIF, PISANTY & STAUB**
**Modiano & Associati Via Meravigli, 16**
**I-20123 Milan(IT)**

(54) **Device for dilating the neck of the uterus for medical use.**

㊼ The device (1) comprises an elastically defor-
mable hollow body (2) which can be inserted in the
neck of a uterus (5). The hollow body (2) is asso-
ciated with a handle (4), wherein a chamber is de-
fined which can be filled with a liquid and which
communicates through a conduit (3) with the inside
of the hollow body (2). The chamber slidably accom-
modates a piston (11), which is controllably operata-
ble for conveying said liquid in the hollow body (2)
for causing dilatation thereof.

FIG. 3

EP 0 265 536 A1

## DEVICE FOR DILATING THE NECK OF THE UTERUS FOR MEDICAL USE

The present invention relates to a device for dilating the neck of the uterus for medical use.

Devices of this kind are known which are generally composed of a series of metallic bodies of increasing diameter, better known in medicine as Hogar dilators, which are introduced individually in the neck of the uterus, left 'in situ' for a short time and are then extracted to be replaced by a metallic body with a large diameter.

These dilators, though they obtain rather positive results, must be used by very expert persons in order to avoid the risks of possible lacerations and/or perforations.

In order to solve this problem, in US patent No. 4.228.801 a dilator is described for applications in the medical field which comprises an elastically deformable hollow body, having such dimensions as to be suitable for being introduced in the neck of a uterus, which dilator can be dilated by pumping pressurised fluid in the hollow body.

Pumping is accomplished by means of a pump connected to a handle which supports the hollow body.

This kind of dilator has proved to be very effective and safe, obtaining a gradual and painless dilatation.

During experimentation, this dilator has proved to be susceptible of further improvements.

In particular, the need has been felt to eliminate the dependance of the dilator from an external pump having rather bulky dimensions, in order to obtain better manoeuvrability of the device during use.

A disadvantage indicated by the dilator described above is that it cannot determine the actual dilation of the neck of the uterus, moment by moment; for this purpose, the dilator had been fitted with a manometer which measured the pressure of the liquid pumped into the elastically deformable body, but the values detected turned out to be scarcely indicative of the dilation, since the same could be biased by the individual characteristics which vary from patient to patient.

The basic aim of the present invention is to eliminate the disadvantages described above by providing a device which is easy to manufacture, highly manoeuvrable and safe in operation.

Within the scope of this aim, an object of the invention is to provide a device which constantly monitors the actual dilation of the neck of the uterus in which the hollow body is inserted during operation.

Another object of the invention is to provide a device which, even in the event of a breakage of the hollow body during use, preserves the patient from injury.

This aim, as well as this and other object which will become apparent hereinafter, are achieved by a device for dilating the neck of the uterus for medical use, comprising an elastically deformable hollow body which can be inserted in the neck of a uterus, said hollow body being associated with a handle, characterized in that in said handle a chamber is defined which can be filled with a liquid and which communicates through a conduit with the inside of said hollow body, said chamber slideably accommodating a piston which is controllably operatable for conveying said liquid in said hollow body, for the dilation thereof.

Further features and advantages will become apparent from the detailed description of a device according to the invention, illustrated by way of example in the accompanying drawings, where:

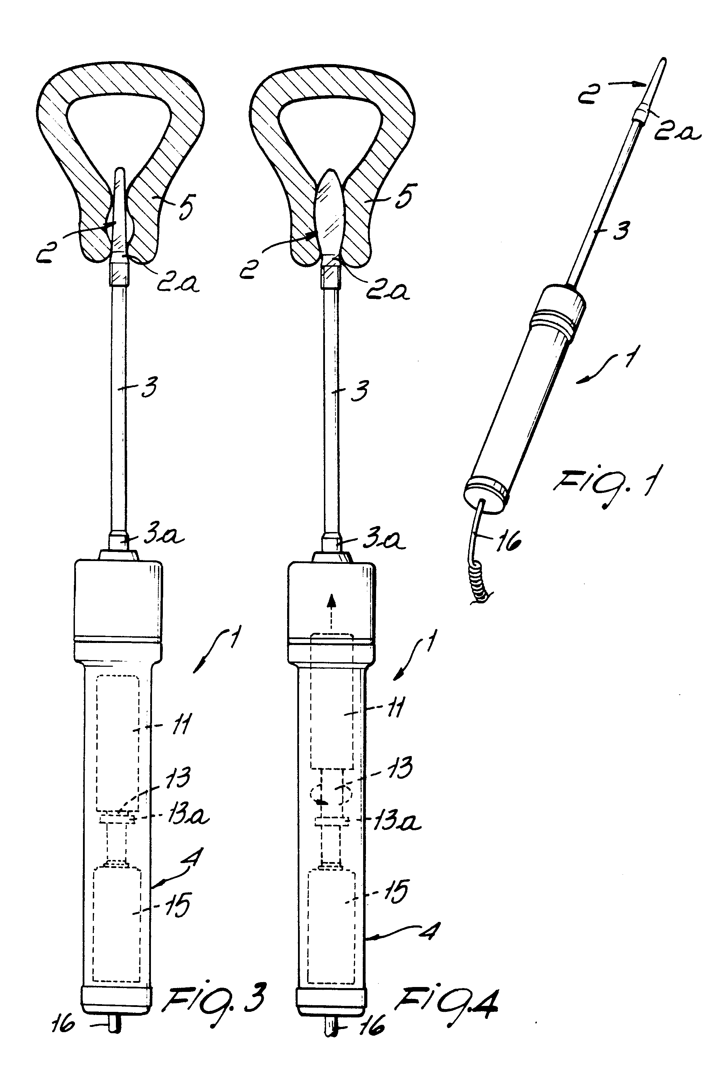

Fig. 1 is a perspective view of the device according to the invention;

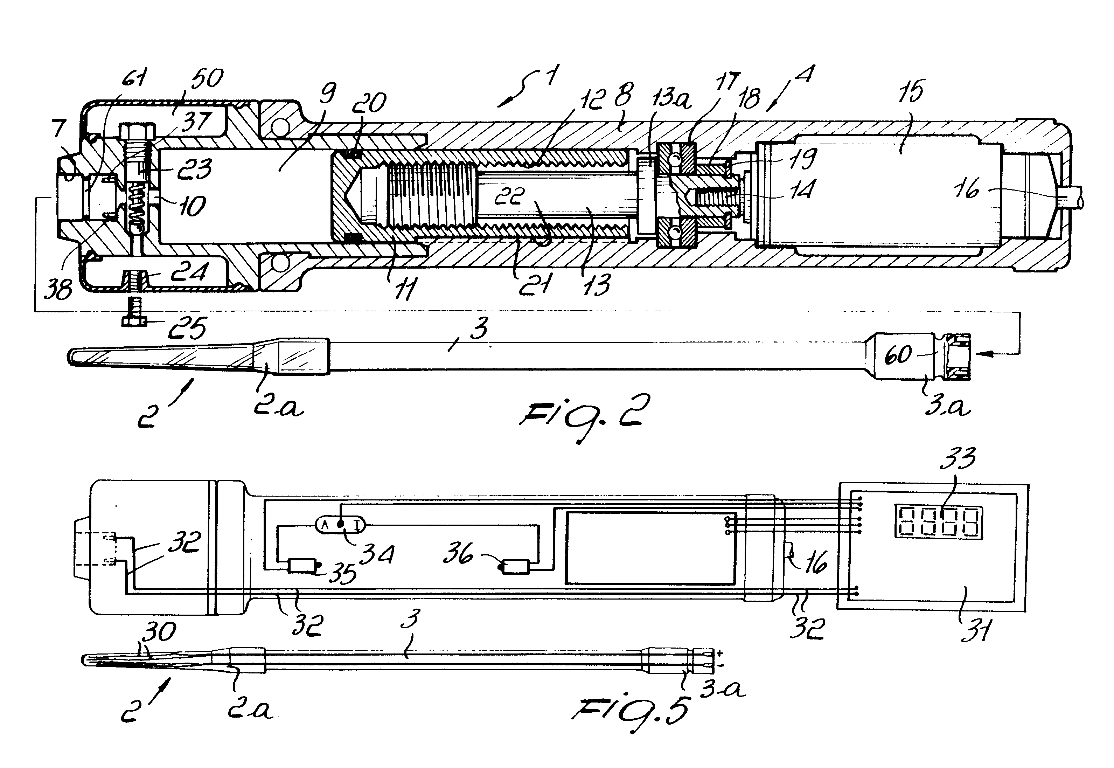

Fig. 2 is an enlarged cross section along the longitudinal axis of the handle, laid adjacent the hollow body of the device according to the invention;

Figs. 3 to 4 are schematic views which point out the use of the device according to the invention; and

Fig. 5 is a schematic view of the control and operating connections provided in the device according to the invention.

With reference to the figures, the device according to the invention, indicated generally with the reference numeral 1, comprises a hollow body 2 provided in an elastically deformable material with high mechanical resistance characteristics and associated through a tubular conduit 3 with a handle 4. The hollow body advantageously has a conical shape to facilitate its insertion in the opening of the neck of a uterus 5 and has, proximate to the base, a supporting widening 2a for making its positioning easier.

The hollow body 2 is rigidly coupled with an end of the tubular conduit 3 which has its other end 3a rigidly associatable by means of a quick-type coupling with the handle 4. For this reason, on the end 3a a recess 60 is provided which extends circumferentially, while on an end of the framework 4 is provided a hole 7, in which the end 3a is insertable, and which is correspondingly provided with an annular protrusion 61 engageable with the recess 60. The handle 4 is essentially composed of a containment body 8 which defines internally, according to the invention, a chamber 9 which, through a connecting conduit 10, communicates with the tubular conduit 3 and then with the hollow body 2. In the chamber 9, which can be filled with a liquid, is slideably accommodated a piston 11 which, by moving with an alternate motion, conveys the liquid in the hollow body 2 giving rise to its gradual dilatation, or returns the liquid from the hollow body 2 to the chamber 9, giving rise to the return of the hollow body to its original dimensions. To allow a very slow motion of the piston 11, a threaded seat 12 has been provided internally and coaxially thereto which engages with a threaded shaft 13, which is operatable with a rotating motion around its axis. More in particular, for the threaded shaft 13 threading can be provided for only a terminal part thereof, by rigidly associating the other end with the output 14 of a reducer or ratiomotor 15, which is in turn accommodated in the containment body 8 of the handle. The reducer 15 can be electrically powered by means of an electrical cable 16 which protrudes from the handle on the opposite side with respect to the hollow body.

The threaded shaft 13 can be rotatably supported by a thrust bearing 17 positioned on the shaft between a shoulder 13a and a spacer 18, locked by an elastic ring 19.

For completeness of description, it should be noted that the threaded shaft 13 can be screwed onto the output of the reducer, while on the piston 11 one or more sealing rings 20 can be provided.

To avoid rotation of the piston 11 around its axis, an extension 21 is provided which extends along the internal wall of the chamber in a longitudinal direction and which slideably engages within a groove 22 defined on the outer skirt of the piston 11.

Transversely to the connecting conduit 10 a one-way valve 23 is provided which communicates the chamber 9 with a loading chamber 50, which extends circumferentially to the chamber 9 and is in communication with the outside through a hole 24 which can be closed with a stopper 25. Through the hole 24 the liquid can be inserted, which, by opening the valve 23, fills the chamber 9.

Suitably, the containment body 8 can be provided by molding with thermoplastic material, such as polyvinylchloride (PVC) and by providing the piston in Teflon to reduce sliding friction.

Advantageously, the device also comprises sensors 30 for detecting the actual deformation of the hollow body 2. These sensors 30 can be composed of a plurality of ramifications in fine wire of low-resistivity material, such as e.g. silver or copper, which are rigidly coupled with the outer surface of the hollow body 2.

These ramifications are traversed by a low-intensity and low-voltage electric current, and are connected to a decoder 31 through the connecting wires 32. The decoder 31 is furthermore connected to a visual display 33 on which the actual dilation of the hollow body 2, and therefore the actual dilatation of the neck of the uterus 5, can be read.

More in particular, on the hollow body 2 an electrically conducting paste-like substance is applied, e.g. an alumina-or copper-based compound, which adheres to the hollow body 2 and provides an electrical connection between the ramifications of the sensors 30. The deformation of the hollow body, i.e. its dilatation, gives rise to an alteration of the resistance opposed by the ramifications and by the paste-like substance which connects them, which causes a variation in the voltage drop which can be detected at the ends of the connecting wires 32 by the decoder 31 and is transformed into the value displayed.

These sensors for detecting the actual deformation of the hollow body 2 can also be composed of an electric straingauge of a known kind, which is accommodated internally to the hollow body 2, which has its opposite ends rigidly associated with two diametrically opposite points of the inner surface of the hollow body, and which is connected with the wires 32. The variation in the strain applied to the ends of the straingauge gives rise to a variation in its resistance, which is detected and visualized by the decoder.

The reducer 15 can be operated by means of an operating button 34 coupled in series with stroke limiting devices 35 and 36 which break the power supply circuit of the reducer 15 when the piston 11 has ended its advancing or return motion, preventing the breakage of the threaded shaft 13 or of the threaded seat 12.

For completeness of description, it should be noted that in order to provide electrical continuity of the connecting wires 32 in the connecting zone between the tubular conduit 3 and the handle 4, connections of the male-female type can be provided, indicated respectively with the numerals 37 and 38, suitably protected to avoid any possible accidental connections between contacts with opposite polarity.

From what has been described, the operation of the device is apparent.

Initially, the filling of the chamber 9 must be performed, with the piston 11 fully retracted, with the liquid, e.g. a physiological solution, through the hole 24; after this operation, the hollow body 2 is fixed, supported by the tubular conduit 3 to the handle 4. After inserting the hollow body 2 into the neck of the uterus 5, the doctor operates, by means of the operating button 34, the reducer 15, which gives rise to the advancement of the piston 11 in the chamber 9. This causes a slow and progressive dilation of the hollow body 2, with a consequent dilation of the neck of the uterus, which is constantly monitored by means of the visual display 33.

At the end of the dilating operation, the doctor reverses the motion of the piston and removes the hollow body.

Before a new operation, it is necessary to replace the hollow body and the tubular conduit with other sterile ones.

In practice it has been observed that the device according to the invention fully achieves the aim proposed, obtaining a high operating reliability together with a great manoeuvrability, due to the fact that the need for an external pump has been eliminated.

Another advantage is to be able to monitor at any time, the actual dilatation obtained, independently of the individual characteristics of the patient.

A further advantage is that of obtaining with a small apparatus relatively high pressures (in the range of 7-8 atmospheres) which are indispensable to overcome the resistance of the cervical duct of the uterus.

Furthermore, the fact that a practically incompressible liquid is used, ensures a high safety factor even in case of an accidental rupture of the hollow body.

Practically, the materials employed, so long as compatible with the specific use, as well as the dimensions, may be any according to requirements and the state of the art.

## Claims

1. Device for dilating the neck of the uterus for medical use, comprising an elastically deformable hollow body which can be inserted in the neck of a uterus, said hollow body being associated with a handle, characterized in that in said handle is defined a chamber, which can be filled with a liquid and communicates through a conduit with the inside of said hollow body, said chamber slideably accommodating a piston, which is controllably operatable for conveying said liquid in said hollow body for the dilatation thereof.

2. Device according to Claim 1, characterized in that in said piston is defined a threaded seat, engaged by a threaded shaft, which is operatable with a rotating motion around its own axis, for the alternate motion of said piston in said chamber, means being provided for preventing rotation of said piston around its own axis.

3. Device according to Claims 1 and 2, characterized in that said means comprise at least one extension, extending internally to said chamber in a direction parallel to the longitudinal axis of said chamber, and slideably engaging in a groove defined on the outer skirt of said piston.

4. Device according to Claims 1 and 2, characterized in that said threaded shaft is rigidly associated with the output of a reducer accommodated in said handle.

5. Device according to Claim 1, characterized in that said chamber communicates through a one-way valve with an opening which can be closed by a stopper, for filling said chamber with said liquid.

6. Device for dilating the neck of the uterus for medical use, comprising an elastically deformable hollow body, which can be inserted in the neck of a uterus, said hollow body being dilatable with pressurized liquid pumped inside said hollow body, characterized in that it comprises deformation sensors associated with said hollow body and connected with a visual display for constantly detecting the extent of the dilatation of said hollow body.

7. Device according to Claim 6, characterized in that said deformation sensors are of the electric kind and are traversed by low-voltage and low-intensity electric current.

8. Device according to Claims 6 and 7, characterized in that said deformation sensors comprise a plurality of ramifications in low-resistivity material, rigidly associated with the external surface of said hollow body and connectable to each other by a film of deformable electroconductive material.

9. Device according to Claims 6 and 7, characterized in that said deformation sensors comprise an electric straingauge, accommodated internally to said hollow body and having the opposite ends rigidly associated with two diametrically opposite points of the surface of said hollow body.

10. Device according to Claim 6, characterized in that said deformation sensors are connected to a decoder with a visual display.

11. Device according to one or more of the preceding claims, characterized in that it comprises stroke limiting devices for said piston for interrupting the rotation of said threaded shaft.

**Fig. 1**

**Fig. 3**

**Fig. 4**

61 50
7
37
23
10
24
38
25
9 20
1 12 8 13a 17 18 4 15
19
14
16
22
11 21 13

**Fig. 2**

2 2a 3 60 3a

32 33
30 32 34 35 36 16 32 31
2 2a 3 3a

**Fig. 5**

0 265 536

# EUROPEAN SEARCH REPORT

**European Patent Office**

Application number

EP 86 11 4865

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 311 560 (ORTHO)<br><br>* Page 7, line 2 - page 8, line 20; figures 5,6 * | 1 | A 61 M 29/02 |
| A | US-A-4 429 724 (DORROS)<br><br>* Description; figures * | 1,2,11 | |
| A | US-A-3 208 388 (GLASGOW)<br><br>* Column 2, line 62 - column 5, line 20; figures * | 2,5 | |
| A | US-A-3 858 581 (KAMEN)<br><br>* Description; figures 1,3 * | 2-4 | |
| | | | TECHNICAL FIELDS SEARCHED (Int. Cl.4)<br><br>A 61 M |

XIXXXXXXXXXXXXXXXXXXXXXXXXXXXXXXXXXXXXXXXXX

| Place of search | Date of completion of the search | Examiner |
|---|---|---|
| The Hague | 29-06-1987 | VANRUNXT |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent
Office

## CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☐ No claims fees have been paid within the prescribed time limit. The present European search report has been drawn up for the first ten claims.

## X LACK OF UNITY OF INVENTION

The Search Division considers that the present European patent application does not comply with the requirement of unity of invention and relates to several inventions or groups of inventions.
namely:

1) Claims 1-5,11: Pumping means incorporated in the handle of a dilating device.

2) Claims 6-10: Deformation sensors.

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☒ None of the further search fees has been paid within the fixed time limit. The present European search report has been drawn up for those parts of the European patent application which relate to the invention first mentioned in the claims,

namely claims: 1-5,11